# EUROPÄISCHE PATENTSCHRIFT

(11) **EP 3 651 686 B1**
(45) Veröffentlichungstag und Bekanntmachung des Hinweises auf die Patenterteilung: **22.11.2023**
(21) Anmeldenummer: 18745514.2
(22) Anmeldetag: 10.07.2018
(51) Int. Cl.: A61F 2/30, A61F 2/28, A61C 13/00

(54) **KNOCHENIMPLANTAT ZUR VERWENDUNG ALS FÜLLSTÜCK BEI DER KORREKTUR EINER FEHLSTELLUNG EINES KNOCHENS IM BEREICH DYSGNATHIE UND HERSTELLUNGSVERFAHREN**
BONE IMPLANT FOR USE AS A GAP FILLER IN THE CORRECTION OF A MALPOSITION OF BONE IN THE DOMAIN OF DYSGNATHIA AND PRODUCTION METHOD
IMPLANT OSSEUX DESTINÉ À ÊTRE UTILISÉ COMME PIÈCE DE REMPLISSAGE LORS DE LA CORRECTION D'UNE MALPOSITION D'UN OS DANS LE DOMAINE DE LA DYSGNATHIE ET PROCÉDÉ POUR SA FABRICATION

(30) Priorität: 10.07.2017 DE 102017115403
(43) Veröffentlichungstag der Anmeldung: 20.05.2020
(73) Patentinhaber: Karl Leibinger Medizintechnik GmbH & Co. KG, 78570 Mühlheim (DE)
(72) Erfinder: SWENNEN, Gwenn, 8300 Knokke (BE); REINAUER, Frank, 78576 Emmingen-Liptingen (DE); WOLFRAM, Tobias, 63303 Dreieich (DE); AKSU, Adem, 78054 VS-Schwenningen (DE)
(74) Vertreter: Winter, Brandl - Partnerschaft mbB
(86) Internationale Anmeldenummer: PCT/EP2018/068659
(87) Internationale Veröffentlichungsnummer: WO 2019/011913

(56) Entgegenhaltungen:
- EP-A1- 2 730 298
- WO-A1-00/59409
- WO-A1-03/030787
- WO-A2-98/12995
- WO-A2-2013/006778
- RU-C1- 2 572 355
- US-A1- 2008 228 278

## Beschreibung

Die Erfindung betrifft ein Knochenimplantat geeignet zur Verwendung als Füllstück zur Korrektur einer Fehlstellung eines Knochens, insbesondere einer Kieferfehlstellung, wobei das Implantat einen ersten Abschnitt zur Befestigung an einem ersten Knochenabschnitt des Knochens und einen zweiten Abschnitt zur Befestigung an einem zweiten Knochenabschnitt des Knochens aufweist, und wobei das Knochenimplantat vorbereitet ist, um im am Knochen fixierten Zustand den ersten Knochenabschnitt und den zweiten Knochenabschnitt zueinander auszurichten und voneinander beabstandet, dadurch ein Volumen vorgebend, zu halten. Weiterhin betrifft die Erfindung ein Herstellungsverfahren für ein solches Knochenimplantat.

Knochenimplantate kommen auch bei sogenannten Dysgnathien zum Einsatz. Unter eines Dysgnathie versteht man eine Fehlentwicklung der Zähne, der Kiefer und/oder des Kausystems, die die Zahnstellung, die Verzahnung, die Kieferform, die Lage der Kiefer zueinander oder den Einbau der Kiefer in den Schädel betreffen. Dadurch entstehen sowohl ästhetische als auch funktionelle Beeinträchtigungen im Kiefer- und Gesichtsbereich. Solche Fehlstellungen können unter anderem zu Fehlbelastungen des Kiefergelenks mit Knack- und Reibegeräuschen, sowie zu Beschwerden wie Schmerzen und Bewegungseinschränkungen führen. Allerdings können die Beschwerden sich auch über den Kiefer- und Gesichtsbereich hinaus erstrecken. Dazu zählen schmerzhafte Verspannungen der Gesichts- und Kaumuskulatur, knirschen oder pressen zur Beseitigung der Zahnfehlstellung oder der Kieferfehlstellung, Beeinträchtigungen der Nasenatmung, Austrocknung der Mundhöhle, Beschwerden in der Hals- und Schultermuskulatur und chronische Rücken- oder Nackenschmerzen. All diese Beschwerden können durch die fehlerhafte Kieferstellung verursacht werden, weswegen es üblich ist, die Kieferfehlstellung durch eine operative Korrektur zu beheben. Die Erfindung ist auf diesem Gebiet angeordnet.

Es gibt mehrere Arten der Dysgnathieformen/Kieferfehlstellungsformen. So können die Kiefer zu weit vorne oder zu weit hinten liegen, nach rechts oder nach links abweichen, oder zu hoch oder zu tief im Gesichtsschädel eingebaut sein. Dabei ist die Unterkieferrücklage, bei der der Unterkiefer relativ zu dem Oberkiefer zurückgesetzt ist, die häufigste Form der Dysgnathie.

Aus dem Stand der Technik ist es bekannt, diese Kieferfehlstellung zu korrigieren, indem in einem operativen Eingriff der Kieferknochen durchtrennt wird und mit kleinen Titanschrauben oder Titanplatten an seiner neuen, gewünschten Stellung fixiert wird. Dazu offenbart die WO 97/01991 A1 eine Vorrichtung zur Fixation von Knochenfragmenten mit einer longitudinalen Knochenplatte, die an ihren beiden Enden mindestens je ein durchgehendes Schraubenloch aufweist, wobei (A) die Knochenplatte ein zentrales, sich in Richtung der Plattenlängsachse erstreckendes Langloch aufweist; (B) ein Gleiter vorgesehen ist, der auf den das Langloch seitlich begrenzenden Stegen in Richtung der Plattenlängsachse verschiebbar angeordnet ist; und (C) der Gleiter ein, im aufgesetzten Zustand, quer zur Plattenlängsachse verlaufendes Langloch aufweist. Bei dieser Vorrichtung wird also der Knochen durchtrennt, die beiden Abschnitte zueinander positioniert und in der gewünschten Position über von außen an den Kiefer angebrachte Platten oder Schrauben befestigt.

Zudem zeigt die WO 00/59409 A1 eine als Abstandshalter dienende Knochenimplantatvorrichtung. Ferner zeigt die RU 2 572 355 C1 eine identisch mit einem herausgeschnittenen Segment des Kieferknochens ausgebildetes individuelles Implantat. Aus der WO 03/03787 A1 ist eine Herstellung von patientenspezifischen Implantaten bekannt. Weiter ist aus der WO 98/12995 A2 ein passgenaues Knochenimplantat bekannt. Aus der US 2008/228278 A1 ist ein Prothesenmodul für einen Unterkieferknochen bekannt. Das Prothesenmodul umfasst einen Körper, der eine dem Unterkieferknochen entsprechende Form aufweist und sich zwischen einem ersten Ende und einem zweiten Ende, das dem ersten Ende gegenüberliegt, erstreckt. Ein erster Schaft erstreckt sich vom Körper am ersten Ende und ist so konfiguriert, dass er sich in einen Unterkieferknochen hinein erstreckt und diesen mit ihm verbindet, um einen resezierten Abschnitt davon zu ersetzen. Am zweiten Ende befindet sich mindestens ein zweiter Schaft, ein Loch oder eine Geometrie, die einem Kondylus-Ramus-Winkelteil eines Unterkieferknochens ähnelt.

Der Stand der Technik hat jedoch immer den Nachteil, dass zum einen eine Fixierung von außen am Knochen im Gesicht sichtbar ist und somit die Ästhetik des Patienten beeinflusst. Zum anderen ist ein zweiter operativer Eingriff notwendig, um die Fixierungsplatte oder die Fixierungsschrauben wieder zu entfernen. Auch bleibt in diesem Bereich der Trennung ene Vertiefung, auch wenn diese von Haut bedeckt ist, zu erkennen. Dies ist ästhetisch äußerst mangelhaft. Als weiterer Nachteil stellt sich dar, dass die Ausrichtung der beiden Knochenabschnitte zueinander während des operativen Eingriffs erfolgt und z. B. über das Langloch eingestellt werden muss. Dadurch hängen der Erfolg der Operation und das ästhetische und funktionelle Ergebnis also stark von dem Können des Operateurs ab.

Es ist also die Aufgabe der Erfindung, die Nachteile aus dem Stand der Technik zu vermeiden oder wenigstens zu verringern. Insbesondere soll ein Knochenimplantat bereitgestellt werden, das ein optimales operatives Ergebnis gewährleistet und einen eventuellen zweiten Eingriff vermeidet, dadurch, dass das Knochenimplantat von dem Körper abgebaut werden kann und in einen natürlichen Knochen übergeht. Es sind zwar bereits Knochenregenerationsprodukte und Knochenersatzmaterialien bekannt, jedoch kommen diese als Granulate, aushärtbare Zemente oder vorgefertigte Formkörper mit einfacher Geometrie vor, so dass sie für den Einsatz als Implantate mit angepasster Form und Strukturierung ungeeignet oder zumindest nur bedingt einsetzbar sind.

Die Aufgabe der Erfindung wird durch ein Knochenimplantat mit den Merkmalen des Anspruchs 1 sowie durch ein Herstellungsverfahren für ein solches Knochenimplantat mit den Merkmalen des nebengeordneten Anspruchs 12 gelöst.

Das Knochenimplantat soll also mehrere Funktionen übernehmen. So soll einerseits ein Spalt während des operativen Verlagerns geschaffen werden und andererseits nachfolgend gehalten werden, um dort autologes, knöchernes Ersatzmaterial im Heilungsprozess positionieren zu können, insbesondere dort hineinwachsen / nachwachsen zu lassen. Dazu ist das Knochenimplantat als Füllstück ausgelegt, dass die Lücke zwischen zwei voneinander (vollständig) getrennten Knochenabschnitten volumenfüllend schließt. Während des Heilungsprozesses wird dieses Füllstück vollständig oder zumindest überwiegend durch körpereigenen Knochen ersetzt, da dieser in das Füllstück, dieses resorbierend, einwächst. Ungewünschte anatomische Abweichungen sollen vermieden werden, beispielsweise Dellen unter der Haut.

Die Aufgabe der Erfindung wird erfindungsgemäß dadurch gelöst, dass das Knochenimplantat eine solche Geometrie besitzt und angepasst ist, so dass das Knochenimplantat zwischen dem ersten Knochenabschnitt und dem zweiten Knochenabschnitt einsetzbar ist, so dass eine vorbestimmte Ausrichtung des zweiten Knochenabschnitts relativ zu dem ersten Knochenabschnitt erzwungen ist. Mit anderen Worten heißt das, dass das Knochenimplantat nicht von außen, also nach außen abstehend, an den Knochenabschnitten angebracht ist, sondern als eine Art Abstandshalter / Gap-Füller / Lückenausfüller / Füllelement / Platzhalter bzw. Knochenkeil ausgebildet ist. Das Knochenimplantat wird also bspw. in der Längsrichtung des Knochens eingeschoben/eingesetzt.

Mit anderen Worten heißt dies auch, dass das Knochenimplantat an einer Stelle eingesetzt ist, so dass es natürlichen Knochen ersetzt, also ein Knochenstück, das die Fehlstellung korrigiert, bildet. Das Knochenimplantat ist also nicht so ausgebildet, dass es eine plattenartige und/oder externe, äußere Fixierung für die beiden Knochenabschnitte darstellt, sondern dass es eine interne Fixierung, die eine ursprüngliche/gewünschte Form des Knochens nachbildet, darstellt. Das erfindungsgemäße Knochenimplantat ist also so ausgebildet, dass der erste und der zweite Knochenabschnitt bei Einsatz des Implantats zwangsausgerichtet werden, also eine Position der beiden Knochenabschnitte nicht mehr manuell, z.B. während der Operation, zwingend nachjustiert werden muss. Natürlich soll für bestimmte Problemfälle eine Feinjustierung aber möglich bleiben. Dies hat den Vorteil, dass bei einer Implantierung des Knochenimplantats, also eine Zwangsausrichtung des ersten Knochenabschnitts und des zweiten Knochenabschnitts bzw. der Position der beiden gegenüber einander, erreicht wird, die einer gewünschten, optimalen Position entspricht. Die Position der beiden Knochenabschnitte muss also nicht mehr zwingend während der Operation eingestellt werden, so dass zum einen ein besseres Ergebnis erzielt werden kann und zum anderen die Operationszeit erheblich verkürzt werden kann. Eine Einstellung kann auf Wunsch aber erfolgen.

Mit anderen Worten wird also ein Platzhalter verwendet, mit dem Positionsinformationen übertragen werden. Dessen mechanische Integrität sorgt für eine stabile Fixierung.

Vorteilhafte Ausführungsformen werden in den Unteransprüchen beansprucht und werden nachfolgend näher erläutert.

Zusätzlich ist es zweckmäßig, wenn der erste Abschnitt zum Anliegen an dem ersten Knochenabschnitt und der zweite Abschnitt zum Anliegen an dem zweiten Knochenabschnitt ausgebildet sind, wobei der erste Abschnitt dem zweiten Abschnitt gegenüberliegend angeordnet ist. Dabei heißt gegenüberliegend, dass der erste Abschnitt an einer ersten Seite/Fläche des Knochenimplantats angeordnet ist, die eine andere Seite/Fläche des Knochenimplantats ist als eine zweite Seite/Fläche, an der der zweite Abschnitt des Knochenimplantats ausgebildet ist, wobei die erste Seite und die zweite Seite diametral zueinander angeordnet sind. Durch die Anlage des Knochenimplantats mit der ersten Seite an dem ersten Knochenabschnitt und mit der zweiten Seite an dem zweiten Knochenabschnitt wird also die Geometrie des Knochenimplantats bestimmt und wie die Knochenabschnitte zueinander verschoben werden. Insbesondere wird dabei zum einen der Abstand (z.B. durch den Abstand der beiden Seite voneinander) zwischen den beiden Knochenabschnitten korrigiert und zum anderen aber auch eine Relativverschiebung (z.B. durch eine Neigung der beiden Seiten zueinander) in der Oben-Unten-Richtung, der Vorne-Hinten-Richtung oder der Rechts-Links-Richtung festgelegt.

Insbesondere ist es von Vorteil, wenn die Geometrie des Knochenimplantats an die individuelle Fehlstellung des Knochens angepasst ist, d. h., einer korrigierenden Geometrie entsprechend, d. h., eine Differenz zwischen der Fehlstellung und einer Sollstellung entsprechend ausgebildet ist. Diese kann auch in einer Überkorrektur bestehen oder diese beinhalten, also derart beschaffen sein, dass das als Füllstück / Platzhalter agierende Knochenimplantat über den Knochen hinaussteht. In diesem Fall kann einem überschießenden Knochenabbau vorgebaut werden. So kann also vorher festgelegt werden, nämlich während der Erstellung des individuellen Knochenimplantats, wie die Kieferfehlstellung korrigiert werden soll.

Ferner ist es bevorzugt, wenn das Knochenimplantat biodegradierbare metallische Werkstoffe enthält oder daraus aufgebaut ist und/oder resorbierbare Polymere oder Keramikwerkstoffe enthält oder daraus aufgebaut ist. Bewährt haben sich insbesondere Magnesium, Magnesiumlegierungen, Eisen, Eisenlegierungen, Barium und Strontium. Bei Polymeren und Keramiken haben sich auch deren Komposite bewährt. Erwähnenswert sind hier insbesondere solche wie PDLLA, PLGA, PLA, PGA, Chitosan-Fasern / -Partikel, HAP, CaCOs; alpha-/beta TCP, Hydroxylapatit und Kalziumphosphat, etwa biphasisches Kalziumphosphat (BCP). Alle biodegradierbaren metallischen Werkstoffe und alle resorbierbaren Polymere, Keramiken und deren Komposite sind grundsätzlich geeignet, haben aber alle spezifische Vorzüge und Besonderheiten. Ca₃(PO₄)₂O₃ ist ebenfalls wie Mixturen aus beta-TCP und Hydroxylapatit geeignet. Auch ist der Einsatz von singulärer Hinzugabe von HA bei den genannten Werkstoffen denkbar.

Von Vorteil ist es auch, wenn das Füllstück Kalzium/Calcium und Phosphat umfasst, da der natürliche Knochen größtenteils aus Kalziumphosphat/Calciumphosphat besteht, wodurch synthetische Kalziumphosphatverbindungen geeignete Knochenersatzmaterialien darstellen.

Zusätzlich ist es von Vorteil, wenn das Knochenimplantat Trikalziumphosphat (Ca₃(PO₄)₂). Insbesondere diese Materialien haben sich als geeignet dargestellt, ein bioresorbierbares Knochenimplantat zu bilden. Trikalziumphosphat wird oftmals als Knochenersatzmaterial und Knochenaufbaumaterial eingesetzt.

Weiter bevorzugt kann das Knochenimplantat β-Trikalziumphosphat (β-TCP) umfassen, da dieses Material geeignete Eigenschaften u.a. hinsichtlich Form, Struktur, Bioresorbierbarkeit, Verträglichkeit und Festigkeit aufweist.

Es ist auch möglich, dass das Knochenimplantat α-Trikalziumphosphat (α-TCP), Hydroxilapatit (HA) und/oder eine Mischung aus β-Trikalziumphosphat (β-TCP) und Hydroxilapatit (Biphasisches Kalziumphosphat, abgekürzt BCP) umfasst.

Es können auch Blutprodukte (PRP's) Zellen und/oder biologisch aktive Moleküle, wie z.B. Proteine, Peptide oder DNA, RNA und/oder Oligonucleotide eingesetzt sein.

Um ein stabiles Einsetzprodukt zu erhalten, hat es sich bewährt, wenn das als Füllstück ausgebildete Knochenimplantat zumindest abschnittsweise einen fachwerkartigen oder gitterstrukturähnlichen Aufbau besitzt. Vorteilhafterweise kann dabei ein geschlossener oder unterbrochener Rand den fachwerkartigen oder gitterstruktrurähnlichen Aufbau außenseitig umgeben.

Wenn die Stege übereinander gefügt sind und/oder nebeneinander gefügt sind, um ein dreidimensionales Gebilde zu schaffen, so kann ein voluminöses , an allen Schnittflächen anliegendes und volumenmäßig passendes Füllstück genutzt werden, dass auch eine initiale Mindestfestigkeit besitzt, dass für einen problemlosen Einsatz prädestiniert ist.

Damit eine gut für ein Knochenwachstum geeignete Struktur eingesetzt werden kann, die aus ausreichend fest ist, ist es zielführend, wenn die Stege schräg zueinander verlaufen, etwa orthogonal, und Berührungs- und Verbindungsbereiche besitzen, an denen ein einmaterialiger Übergang von einem Steg zum anderen und/oder zum Rand geschaffen ist.

Es ist von Vorteil, wenn zwischen den Stegen wenigstens ein Hohlraum ausgebildet ist.

Ein vorteilhaftes Ausführungsbeispiel ist auch dadurch gekennzeichnet, dass eine Vielzahl von Hohlräumen in vordefinierten Bereichen in Beabstandung zu den Stegen vorhanden ist.

Ein Steg kann dabei so ausgebildet sein, dass er ein Loch mit vorzugsweise geschlossener Lochwandung definiert. Am Steg kann alternativ oder zusätzlich auch ein Haltegriff für den Operateur vorhanden sein, etwa nach Art eines Stifts, eines Zylinders. Dabei kann in dem Griff eine Sollbruchstelle vorhanden sein, vorzugsweise nach der Oberfläche des Füllstücks, insbesondere eines Außenseitig vorhandenen Steges. Die Stege können strangartig oder als Stränge verlaufen. Sie können gerade sein, oder gebogen sein. Auch ist ein Zickzack-Muster denkbar. Im Falle einer Biegung bzw. einer Krümmung kann eine gleichbleibende oder über Länge zunehmende / abnehmende Biegung / Krümmung realisiert werden, was Festigkeitsvorteile bringen kann.

Ein Steg kann nach Art einer dichten Kugelpackung aufgebaut sein, wobei die benachbarten Hohlräume durch das Fehlen von "Kugeln" geprägt sind. Dabei kann der Steg durch Partikel / Pulver / Granulate / Brocken und Anteile daraus mit einer Größe / einem (mittleren) Durchmesser von 1 µm bis 100 µm aufgebaut sein.

Weiterhin ist es vorteilhaft, wenn das Knochenimplantat als massives Implantat, d. h. als ein festes, solides Implantat, das im Wesentlichen ohne Aussparungen und Lufteinschlüsse gebildet ist, und/oder als ein (mikro- / makro) strukturiertes bzw. poröses Implantat, d. h., ein Implantat, das eine Gitterstruktur und/oder viele Aussparungen und/oder Lufteinschlüsse aufweist, ausgebildet ist. Je nach Anwendungszweck können so verschiedene Vorteile für das Knochenimplantat bereitgestellt werden. Insbesondere kann dadurch die Resoptionsgeschwindigkeit / die Festigkeit und das Gewicht des Knochenimplantats geeignet beeinflusst werden.

Die Aussparungen und die Gitterstrukturen / Stege / Stränge ermöglichen gleichzeitig die Aufnahme blutbasierter Produkte und/oder Proteine, wie z.B. von Wachstumsfaktoren und/oder zellulären Systemen, wie z.B. antologe / analoge oder heterologe Zellen / Stammzellen. Aufgrund der besonderen hydrophylen Eigenschaft des produzierten Implantats kommt es hierbei zu einer schnellen Reaktion zwischen diesen Komponenten und der Implantatstruktur, was später zu einer Verbesserung der klinischen Resultate führt.

Ferner ist es zweckmäßig, wenn das Knochenimplantat aus zusammengesetzten Schichten aufgebaut ist und/oder in einem generativen Fertigungsverfahren hergestellt ist. So lässt sich das individuelle Knochenimplantat besonders schnell erzeugen und anschließend zu einem zusammengesetzten Knochenimplantat zusammenfügen. Dabei wird die Funktion des Knochenimplantats nicht beeinträchtigt.

Ein bevorzugtes Ausführungsbeispiel zeichnet sich dadurch aus, dass das Knochenimplantat eine organische Matrix mit keramischen Partikeln (Schlicker) umfasst.

Insbesondere ist es bevorzugt, wenn die Partikel eine homogene Dispersion bilden, d. h., dass sie gleichmäßig verteilt in der Matrix sind. So weist das Knochenimplantat über die gesamte Form die gleichen oder ähnliche strukturelle Eigenschaften auf.

Auch kann die organische Matrix mit den keramischen Partikeln (zwischen 1 und 100 µm) z. B. durch selektive Belichtung, wie einem Digital-Leit-Processing-Verfahren (DLP-Verfahren), ausgehärtet werden. So wird eine besonders feste Struktur des Knochenimplantats erzeugt. Es ist auch möglich, ein anderes Wärmebehandlungsverfahren zur Festigkeitssteigerung einzusetzen.

Auch ist es von Vorteil, wenn die einzelnen Schichten ausgehärtet werden, da sich so eine besonders gleichmäßige Härteverteilung ergibt und der Härteprozess so beschleunigt ablaufen kann.

Ferner ist es von Vorteil, wenn das Knochenimplantat in einem generativen Fertigungsverfahren hergestellt ist. Dadurch kann das Knochenimplantat auf Basis eines errechneten Modells individuell erzeugt werden. Da in einem generativen / additiven Verfahren, z. B. 3D-Druck, Lasersintern, Schichtbautechnologien, ein Implantat direkt aus einem Rechnermodell/Computermodell hergestellt werden kann, wird vermieden, dass für jeden Patienten, also jedes individuelle Knochenimplantat, eine eigene Form oder dergleichen hergestellt werden muss.

Die Erfindung betrifft auch ein Herstellungsverfahren eines erfindungsgemäßen Knochenimplantats, wobei in einem Schritt die Kieferanatomie/Knochenanatomie des Patienten erfasst wird, wobei dann in einem Schnitt eine Geometrie des Knochenimplantats zur Korrektur einer Fehlstellung eines Knochens berechnet wird und generativ / additiv gefertigt wird. Bevorzugt wird das ausgehärtete Knochenimplantat beim Aushärten Stück für Stück aus dem Erschaffungsbett gehoben, wobei neue Partikel in einem Fluid unter das schon teilausgehärtete Knochenimplantat nachgeführt werden und dort durch Bestrahlung, etwa durch einen Laser, wiederum aushärten.

Die Erfindung wird nachfolgend mit Hilfe von Zeichnungen erläutert. Es zeigen:
Fig. 1 eine perspektivische Ansicht eines Knochenimplantats in einem ersten Ausführungsbeispiel, welches nicht gemäß der beanspruchten Erfindung ist,
Fig. 2 eine perspektivische Ansicht eines Knochenimplantats in einem zweiten Ausführungsbeispiel, welches gemäß der beanspruchten Erfindung ist,
Fig. 3 eine perspektivische Darstellung des Knochenimplantats in dem ersten Ausführungsbeispiel im implantierten Zustand in Verbindung mit einem Unterkiefer,
Fig. 4 eine perspektivische Ansicht des Knochenimplantats im dem zweiten Ausführungsbeispiel im implantierten Zustand in Verbindung mit dem Unterkiefer, und
Fig. 5 eine schematische Darstellung der Verfahrensschritte eines Herstellungsverfahren des Knochenimplantats.

Die Figuren sind lediglich schematischer Natur und dienen ausschließlich dem Verständnis der Erfindung. Dieselben Elemente sind mit denselben Bezugszeichen gekennzeichnet. Die unterschiedlichen Merkmale der einzelnen Ausführungsbeispiele können beliebig untereinander ausgetauscht werden.

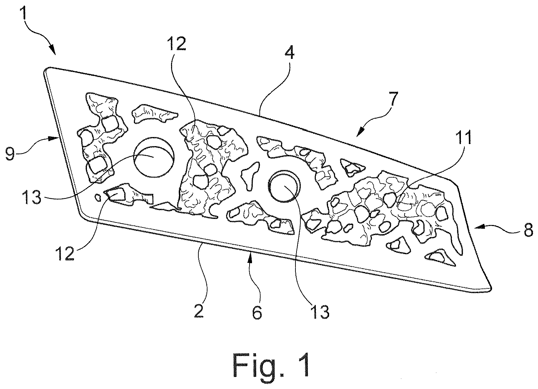

Fig. 1 zeigt ein Knochenimplantat 1, das als Füllstück zum Auffüllen einer Lücke im Knochen bei einer Korrektur einer Fehlstellung eines Knochens, insbesondere einer Kieferfehlstellung, im Bereich der Dysgnathie eingesetzt wird. Das Knochenimplantat 1 weist einen ersten Abschnitt 2 zur Befestigung an einem ersten Knochenabschnitt 3 des Knochens und einen zweiten Abschnitt 4 zur Befestigung an einem zweiten Knochenabschnitt 5 des Knochens auf. Das Knochenimplantat 1 ist vorbereitet, um im am Knochen fixierten Zustand den ersten Knochenabschnitt 3 und den zweiten Knochenabschnitt 5 zueinander auszurichten und voneinander beabstandet zu halten. Das Knochenimplantat 1 besitzt also eine solche Geometrie, die angepasst ist, so dass das Knochenimplantat 1 zwischen dem ersten Knochenabschnitt 3 und dem zweiten Knochenabschnitt 5 eingesetzt werden kann. Beim Einsatz des Knochenimplantats 1 wird also eine vorbestimmte Ausrichtung des zweiten Knochenabschnitts 5 relativ zu dem ersten Knochenabschnitt 3 erzwungen.

Der erste Abschnitt 2 des Knochenimplantats 1 ist an einer ersten Fläche 6 des Knochenimplantats ausgebildet und der zweite Abschnitt 4 des Knochenimplantats 1 ist an einer zweiten Fläche 7 ausgebildet. Dabei sind die beiden Flächen 6, 7 einander gegenüberliegend angeordnet. Ferner weist das Knochenimplantat 1 an einer Unterseite eine Fläche 8, und an einer Oberseite eine Fläche 9 auf, die sich im eingesetzten Zustand in die Kontur des Knochens einpassen. Das Knochenimplantat/Implantat 1 aus Fig. 1 weist eine im Wesentlichen quaderförmige Form auf. Jedoch kann die Form des Implantats 1 je nach Knochenfehlstellung stark variieren.

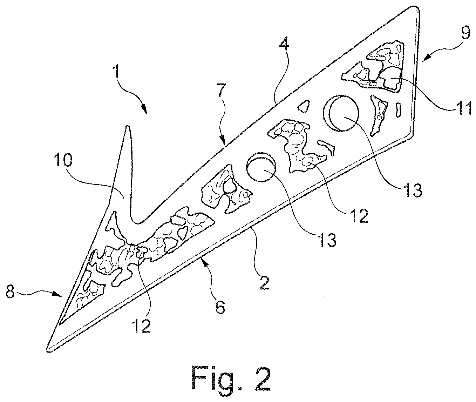

Das Implantat 1 aus Fig. 2 weist die Form einer "1" oder die Form eines Hakens mit einem an der zweiten Seite 7 hervorstehenden, spitzen Vorsprung 10 auf. Dabei liegt der zweite Knochenabschnitt 5 im eingesetzten Zustand an der zweiten Seite 7 und an der Innenseite des Vorsprungs 10 an. Die Außenseite des Vorsprungs 10 wird durch die Fläche 8 gebildet.

Sowohl bei dem Implantat 1 aus Fig. 1 als auch bei dem Implantat 1 aus Fig. 2 sind die erste Fläche 6 und die zweite Fläche 7 zueinander geneigt, so dass durch den Einsatz des Implantats 1 sowohl eine Beabstandung der beiden Knochenabschnitte 3, 5 als auch eine Relativdrehung erreicht wird.

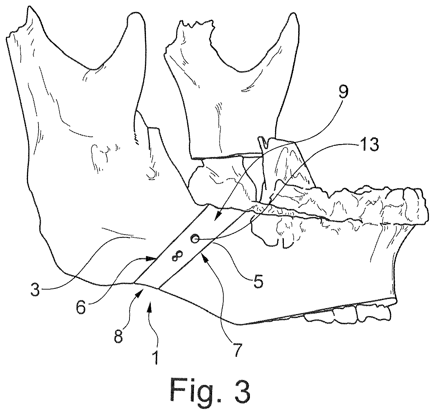

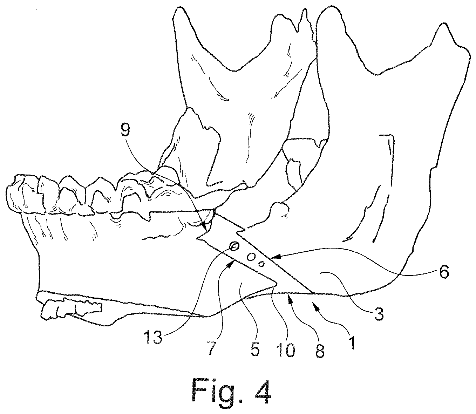

In Fig. 3 bzw. Fig. 4 sind die Implantate aus Fig. 1 bzw. Fig. 2 in einem im Kiefer eingesetzten Zustand dargestellt. Die erste bzw. die zweite Fläche 6, 7 des Knochenimplantats 1 liegt bündig an dem ersten bzw. zweiten Knochenabschnitt 3, 5 an. Das Knochenimplantat 1 kann mit einer Gitterstruktur 11 (vergleiche Fig. 1 oder Fig. 2) ausgebildet sein, so dass es eine Art poröses Implantat mit mehreren Aussparungen 12 bildet, oder als ein massives Implantat (vergleiche Fig. 3 oder Fig. 4) ausgebildet sein.

Das Knochenimplantat weist mehrere Aussparungen nach Art einer Durchgangsbohrung 13 auf, die zur Fixierung durch nicht dargestellte Befestigungsvorrichtungen verwendet werden. Dabei sind die Durchgangsbohrungen 13 in einer Richtung ausgebildet, die senkrecht zu der Längsrichtung des Knochenimplantats 1, also auch senkrecht zu der Längsrichtung des Knochens, ist.

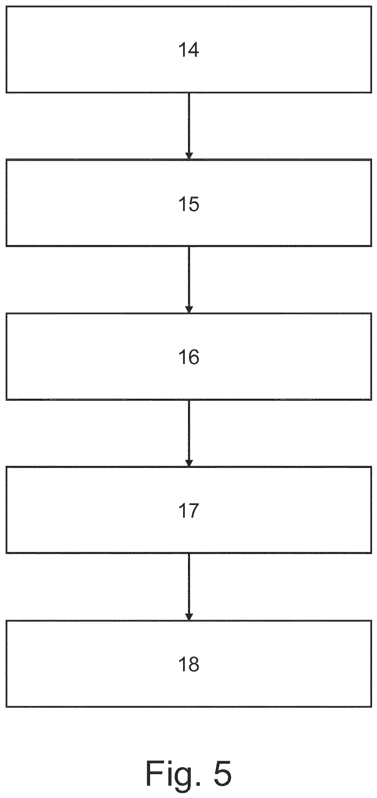

Fig. 5 zeigt die Verfahrensschritte eines Herstellungsverfahrens für ein solches Knochenimplantat 1. Dabei wird in einem ersten Schritt 14 die Kieferanatomie/Knochenanatomie eines Patienten z.B. durch Computertomographie erfasst. In einem zweiten Schritt 15 wird eine Geometrie des Knochenimplantats 1 zur Korrektur der Fehlstellung des Knochens berechnet. Dabei wird unter anderem eine Schnittlinie zum Trennen des Knochens festgelegt und die Geometrie, insbesondere die des ersten Abschnitts 2 und die des zweiten Abschnitts 4, wird an die Schnittlinie angepasst. Anschließend wird die Geometrie des (errechneten) Knochenimplantats 1 in einem dritten Schritt 16 in sehr dünne und definierte Schichten zerlegt. In einem nachgelagerten vierten Schritt 17 werden die Schichten, erfindungsgemäß in einem generativen Fertigungsverfahren erzeugt und optional zusätzlich durch eine Wärmebehandlung in ihrer Festigkeit gesteigert. Danach werden die Schichten in einem fünften Schritt 18 aufeinander gestapelt und miteinander verbunden, so dass ein Knochenimplantat entsteht.

### Bezugszeichenliste

- 1: Knochenimplantat/Implantat
- 2: erster Abschnitt
- 3: erster Knochenabschnitt
- 4: zweiter Abschnitt
- 5: zweiter Knochenabschnitt
- 6: erste Fläche
- 7: zweite Fläche
- 8: Fläche
- 9: Fläche
- 10: Vorsprung
- 11: Gitterstruktur
- 12: Aussparung
- 13: Durchgangsloch
- 14: erster Schritt
- 15: zweiter Schritt
- 16: dritter Schritt
- 17: vierter Schritt
- 18: fünfter Schritt

## Patentansprüche

1. Knochenimplantat (1) zur Verwendung als Füllstück bei der Korrektur einer Fehlstellung eines Knochens im Bereich Dysgnathie,
wobei der Knochen einen ersten Knochenabschnitt (3) und einen zweiten Knochenabschnitt (5) aufweist, die über einen Schnitt voneinander getrennt wurden und über einen Spalt beabstandet sind,
wobei das Knochenimplantat (1) einen ersten Abschnitt (2) zur Befestigung an dem ersten Knochenabschnitt (3) des Knochens und einen zweiten Abschnitt (4) zur Befestigung an dem zweiten Knochenabschnitt (5) des Knochens aufweist,
wobei das Knochenimplantat (1) vorbereitet ist, um im am Knochen fixierten Zustand den ersten Knochenabschnitt (3) und den zweiten Knochenabschnitt (5) zueinander auszurichten und voneinander beabstandet zu halten,
wobei das Knochenimplantat (1) eine solche Geometrie besitzt und angepasst ist, so dass das Knochenimplantat (1) in den Spalt zwischen dem ersten Knochenabschnitt (3) und dem zweiten Knochenabschnitt (5) einsetzbar ist, so dass eine vorbestimmte Ausrichtung des zweiten Knochenabschnitts (5) relativ zu dem ersten Knochenabschnitt (3) erzwungen werden kann,
wobei der erste Abschnitt (2) zum Anliegen an dem ersten Knochenabschnitt (3) und der zweite Abschnitt (4) zum Anliegen an dem zweiten Knochenabschnitt (5) ausgebildet ist, wobei der erste Abschnitt (2) dem zweiten Abschnitt (4) gegenüberliegend angeordnet ist,
wobei der erste Abschnitt (2) an einer ersten Fläche (6) des Knochenimplantats (1) ausgebildet ist und der zweite Abschnitt (4) an einer zweiten Fläche (7) des Knochenimplantats (1) ausgebildet ist,
wobei das Knochenimplantat (1) an einer Unterseite eine dritte Fläche (8) und an einer Oberseite eine vierte Fläche (9) aufweist, die dazu gebildet sind, sich im eingesetzten Zustand in die Kontur des Knochens einzupassen,
wobei die erste Fläche (6) und die zweite Fläche (7) zueinander geneigt sind, so dass durch den Einsatz des Knochenimplantats (1) sowohl eine Beabstandung der beiden Knochenabschnitte (3, 5) als auch eine Relativdrehung erreicht werden kann,
wobei das Knochenimplantat (1) die Form einer "1" oder die Form eines Hakens mit einem an der zweiten Fläche (7) hervorstehenden, spitzen Vorsprung (10) aufweist, wobei der Vorsprung (10) eine Innenseite und eine Außenseite aufweist,
wobei die Außenseite des Vorsprungs (10) durch die dritte Fläche (8) gebildet wird, wobei das Knochenimplantat (1) dazu ausgebildet ist, dass der zweite Knochenabschnitt (5) im eingesetzten Zustand an der zweiten Fläche (7) und an der Innenseite des Vorsprungs (10) anliegen kann.

2. Knochenimplantat (1) nach Anspruch 1, **dadurch gekennzeichnet, dass** das Knochenimplantat (1) biodegradierbare metallische Werkstoffe enthält oder daraus aufgebaut ist und/oder resorbierbare Polymere oder Keramikwerkstoffe enthält oder daraus aufgebaut ist.

3. Knochenimplantat (1) nach Anspruch 1 oder 2, **dadurch gekennzeichnet, dass** das als Füllstück ausgebildete Knochenimplantat (1) zumindest abschnittsweise einen fachwerkartigen oder gitterstrukturähnlichen Aufbau besitzt.

4. Knochenimplantat (1) nach einem der Ansprüche 1 bis 3, **dadurch gekennzeichnet, dass** Stege übereinander gefügt sind und/oder nebeneinander gefügt sind, um ein dreidimensionales Gebilde zu schaffen.

5. Knochenimplantat (1) nach Anspruch 4, **dadurch gekennzeichnet, dass** die Stege schräg zueinander verlaufen und Berührungs- und Verbindungsbereiche besitzen.

6. Knochenimplantat (1) nach einem der Ansprüche 4 oder 5, **dadurch gekennzeichnet, dass** das zwischen den Stegen wenigstens ein Hohlraum ausgebildet ist.

7. Knochenimplantat (1) nach einem der Ansprüche 4 bis 6, **dadurch gekennzeichnet, dass** eine Vielzahl von Hohlräumen in vordefinierten Bereichen in Beabstandung zu den Stegen vorhanden ist.

8. Knochenimplantat (1) nach einem der Ansprüche 4 bis 7, **dadurch gekennzeichnet, dass** einer der Stege so ausgebildet ist, dass er ein Loch mit geschlossener Lochwandung definiert.

9. Knochenimplantat (1) nach Anspruch 8, **dadurch gekennzeichnet, dass** das Loch als eine Durchgangsbohrung ausgebildet ist, die sich im Wesentlichen senkrecht zu der Längsrichtung des Knochenimplantats (1) erstreckt.

10. Knochenimplantat (1) nach Anspruch 8 oder 9, **dadurch gekennzeichnet, dass** an dem Steg ein Haltegriff für einen Operateur angebracht ist, wobei eine Sollbruchstelle zwischen dem Steg und dem Haltegriff ausgebildet ist.

11. Knochenimplantat (1) nach einem der Ansprüche 1 bis 10, **dadurch gekennzeichnet, dass** das Knochenimplantat (1) aus zusammengesetzten Schichten aufgebaut ist und/oder in einem generativen Fertigungsverfahren hergestellt ist.

12. Herstellungsverfahren eines Knochenimplantats (1) nach einem der Ansprüche 1 bis 11, wobei in einem Schritt eine Geometrie des Knochenimplantats (1) zur Korrektur einer Fehlstellung eines Knochens berechnet wird und generativ gefertigt wird.

## Claims

1. A bone implant (1) for use as a filler for correcting an incorrect position of a bone in the field of dysgnathia,
wherein the bone has a first bone portion (3) and a second bone portion (5) that have been separated from each other by a cut and are spaced apart by a gap, the bone implant (1) having a first portion (2) for attachment to the first bone portion (3) of the bone and a second portion (4) for attachment to the second bone portion (5) of the bone,
the bone implant (1) being prepared so that, when fixed to the bone, it orients the first bone portion (3) and the second bone portion (5) relative to each other and keeps said portions at a distance from each other,
the bone implant (1) having a geometry and being adapted such that the bone implant (1) can be inserted in the gap between the first bone portion (3) and the second bone portion (5) so forcing a predetermined orientation of the second bone portion (5) relative to the first bone portion (3) is possible,
wherein the first portion (2) is configured for abutting the first bone portion (3) and the second portion (4) is configured for abutting the second bone portion (5), the first portion (2) being arranged opposite the second portion (4), wherein the first portion (2) is formed on a first surface (6) of the bone implant (1) and the second portion (4) is formed on a second surface (7) of the bone implant (1),
wherein the bone implant (1) has a third surface (8) on a lower side and a fourth surface (9) on an upper side, which are configured to fit into the contour of the bone when inserted,
wherein the first surface (6) and the second surface (7) are inclined to each other, so that by inserting the bone implant (1) both spacing of the two bone portions (3, 5) and a relative rotation can be achieved,
wherein the bone implant (1) has the shape of a '1' or the shape of a hook with a pointed projection (10) protruding from the second surface (7), the projection (10) having an inner side and an outer side,
wherein the outer side of the projection (10) is formed by the third surface (8),
wherein the bone implant (1) is configured to allow the second bone portion (5) to abut the second surface (7) and the inner side of the projection (10) in the inserted state.

2. The bone implant (1) according to claim 1, **characterized in that** the bone implant (1) contains or is made up from biodegradable metallic materials and/or contains or is made up from resorbable polymers or ceramic materials.

3. The bone implant (1) according to any one of the claim 1 or 2, **characterized in that** the bone implant (1) in the form of a filler at least in portions has a truss-type of grid-type structure.

4. The bone implant (1) according to any one of the claims 1 to 3, **characterized in that** webs are joined on top of one another and/or are joined next to one another to create a three-dimensional structure.

5. The bone implant (1) according to claim 4, **characterized in that** the webs extend to be inclined relative to one another and have contacting and connecting areas.

6. The bone implant (1) according to any one of the claims 4 or 5, **characterized in that** at least one cavity is formed between the webs.

7. The bone implant (1) according to any one of the claims 4 to 6, **characterized in that** a plurality of cavities is present in predefined areas at a distance from the webs.

8. The bone implant (1) according to any one of claims 4 to 7, **characterized in that** one of the webs is formed to define a hole with a closed hole wall.

9. The bone implant (1) according to claim 8, **characterized in that** the hole is formed as a through hole extending substantially perpendicular to the longitudinal direction of the bone implant (1).

10. The bone implant (1) according to claim 8 or 9, **characterized in that** a holding handle for an operator is attached to the web, wherein a predetermined breaking point is formed between the web and the holding handle.

11. The bone implant (1) according to any one of the claims 1 to 10, **characterized in that** the bone implant (1) is constructed of composite layers and/or is produced in a generative manufacturing method.

12. A method of producing a bone implant (1) according to any one of the claims 1 to 11, wherein a geometry of the bone implant (1) is calculated in one step for correcting an incorrect position of a bone and is generatively produced.

## Revendications

1. Implant osseux (1) pour l'utilisation comme pièce de remplissage lors de la correction d'un désalignement d'un os dans la zone de la dysgnathie,
dans lequel l'os présente une première section d'os (3) et une seconde section d'os (5) qui ont été séparées l'une de l'autre par une coupe coupe et sont espacées via une fente,
dans lequel l'implant osseux (1) présente une première section (2) pour la fixation à la première section d'os (3) de l'os et une seconde section (4) pour la fixation à la seconde section d'os (5) de l'os, dans lequel l'implant osseux (1) est préparé afin d'orienter, dans l'état fixé à l'os, la première section d'os (3) et la seconde section d'os (5) l'une par rapport à l'autre et de les maintenir à distance l'une de l'autre,
dans lequel l'implant osseux (1) possède une telle géométrie et est adapté de sorte que l'implant osseux (1) puisse être inséré dans la fente entre la première section d'os (3) et la seconde section d'os (5), de sorte qu'une orientation prédéterminée de la seconde section d'os (5) puisse être forcée par rapport à la première section d'os (3),
dans lequel la première section (2) est réalisée pour reposer contre la première section d'os (3) et la seconde section (4) est réalisée pour reposer contre la seconde section d'os (5), dans lequel la première section (2) est agencée à l'opposé de la seconde section (4),
dans lequel la première section (2) est réalisée au niveau d'une première surface (6) de l'implant osseux (1) et la seconde section (4) est réalisée au niveau d'une deuxième surface (7) de l'implant osseux (1),
dans lequel l'implant osseux (1) présente au niveau d'un côté inférieur une troisième surface (8) et au niveau d'un côté supérieur une quatrième surface (9) qui sont formées afin de s'emboîter dans le contour de l'os à l'état inséré,
dans lequel la première surface (6) et la deuxième surface (7) sont inclinées l'une par rapport à l'autre de sorte que, par l'insertion de l'implant osseux (1), non seulement un espacement des deux sections d'os (3, 5) mais aussi une rotation relative puissent être obtenus, dans lequel l'implant osseux (1) présente la forme d'un "1" ou la forme d'un crochet avec une saillie (10) pointue dépassant au niveau de la seconde surface (7),
dans lequel la saillie (10) présente un côté intérieur et un côté extérieur,
dans lequel le côté extérieur de la saillie (10) est formé par la troisième surface (8), dans lequel l'implant osseux (1) est réalisé afin que la seconde section d'os (5) puisse reposer dans l'état inséré contre la seconde surface (7) et contre le côté intérieur de la saillie (10).

2. Implant osseux (1) selon la revendication 1, **caractérisé en ce que** l'implant osseux (1) contient des matériaux métalliques biodégradables ou en est constitué et/ou contient des polymères ou matériaux céramiques résorbables ou en est constitué.

3. Implant osseux (1) selon la revendication 1 ou 2, **caractérisé en ce que** l'implant osseux (1) réalisé comme pièce de remplissage possède au moins par sections une structure en treillis ou similaire à une structure réticulaire.

4. Implant osseux (1) selon l'une des revendications 1 à 3, **caractérisé en ce que** des nervures sont assemblées les unes au-dessus des autres et/ou sont assemblées les unes à côté des autres afin de créer une structure en trois dimensions.

5. Implant osseux (1) selon la revendication 4, **caractérisé en ce que** les nervures s'étendent en biais les unes par rapport aux autres et possèdent des zones de contact et de liaison.

6. Implant osseux (1) selon l'une des revendications 4 ou 5, **caractérisé en ce qu'**au moins un espace creux est réalisé entre les nervures.

7. Implant osseux (1) selon l'une des revendications 4 à 6, **caractérisé en ce qu'**une pluralité d'espaces creux sont présents dans des zones prédéfinies à distance des nervures.

8. Implant osseux (1) selon l'une des revendications 4 à 7, **caractérisé en ce qu'**une des nervures est réalisée de sorte qu'elle définisse un trou avec une paroi de trou fermée.

9. Implant osseux (1) selon la revendication 8, **caractérisé en ce que** le trou est réalisé comme un perçage traversant qui s'étend sensiblement perpendiculairement au sens longitudinal de l'implant osseux (1).

10. Implant osseux (1) selon la revendication 8 ou 9, **caractérisé en ce qu'**une poignée pour un opérateur est montée au niveau de la nervure, dans lequel un point de rupture de consigne est réalisé entre la nervure et la poignée.

11. Implant osseux (1) selon l'une des revendications 1 à 10, **caractérisé en ce que** l'implant osseux (1) est constitué de couches composées et/ou est fabriqué dans un procédé de fabrication additive.

12. Procédé de fabrication d'un implant osseux (1) selon l'une des revendications 1 à 11, dans lequel dans une étape, une géométrie de l'implant osseux (1) est calculée pour la correction d'un désalignement d'un os et est fabriquée de manière additive.
